# DEMANDE DE BREVET EUROPEEN

(11) **EP 4 205 758 A1**
(43) Date de publication de la demande: **05.07.2023**
(21) Numéro de dépôt: 22216892.4
(22) Date de dépôt: 28.12.2022
(51) Int. Cl.: A61K 38/44, A61K 8/66, A61K 8/73, A61K 36/54, A61K 36/81, A61Q 19/00

(54) **SUPEROXYDE DISMUTASE POUR LE TRAITEMENT ET/OU LA PREVENTION DE LA REAPPARITION DES VERGETURES ROSEES**

(30) Priorité: 29.12.2021 FR 2114637
(71) Demandeur: LSI Silderma Ltd, Cork T23AT2P (IE)
(72) Inventeur: DIEHL, Christian, 5008 Córdoba (AR); CHAMI DE DIEHL, Silvia, 5008 Córdoba (AR)
(74) Mandataire: Cabinet Laurent & Charras

(57) **Abrégé**

Superoxyde dismutase, pour utilisation dans le traitement et/ou la prévention de la réapparition des vergetures rosées.

## Description

### Domaine technique

La présente invention se rapporte au domaine technique du traitement et/ou de la prévention des vergetures chez la femme ou l'homme.

Cette composition se trouve être particulièrement efficace pour le traitement et/ou la prévention de la réapparition des vergetures striaes rubrae, dites vergetures rosées.

La présente invention, concerne également un procédé cosmétique d'application de ladite composition pour le traitement et/ou la prévention de la réapparition des vergetures rosées.

### Art antérieur

Les vergetures, également connues sous le nom de striae distensae, striae atrophicae ou encore striae rubrae, sont un type d'atrophie de la peau caractérisé par des dépressions linéaires, bien définies, de nature généralement asymptomatique, particulièrement répandues chez les individus de sexe féminin.

Les vergetures peuvent apparaître sur n'importe quelle partie du corps, à l'exception du visage et des extrémités. Elles apparaissent généralement de façon bilatérale. Elles se forment principalement dans les zones soumises à une tension rapide de la peau sur une courte période, à la suite de lacérations du tissu conjonctif du derme. La région la plus fréquemment touchée par les vergetures est la région abdominale ou la disposition est variable car la direction de la tension cutanée maximale n'est pas constante et peut subir des modifications pour diverses raisons telles que la pré et la post grossesse ; sur les hanches, elles sont souvent transversales ; sur les cuisses, elles sont plus ou moins obliques par rapport à la surface interne. Elles apparaissent aussi fréquemment sur la poitrine.

Les vergetures se présentent généralement comme des bandes linéaires, séparées par des zones de peau saine, avec des bords distincts. Elles ont une longueur de plusieurs centimètres et une largeur de 3 à 10 millimètres, voire plus.

Leur apparition est généralement asymptomatique, même si elle peut parfois s'accompagner de légères démangeaisons. La couleur des vergetures dépend de leurs phases de développement. En effet, lors de la phase inflammatoire, la couleur de la vergeture varie du rouge au violet rosé ou encore au bleu rougeâtre, alors qu'ensuite, durant la phase cicatricielle, les stries sont plus fines, incurvées et deviennent blanchâtres.

Ainsi, on distingue cliniquement deux formes physiologiques de vergetures : les vergetures rosées et les vergetures blanches. En effet, la couleur de la vergeture rosée s'explique par une augmentation de la micro-vascularisation et un processus inflammatoire. Histologiquement, la vergeture rosée présente un oedème dermique avec une prédominance de fines fibres élastiques dermiques, ayant pour conséquence d'amincir le derme. Biochimiquement, la vergeture rosée se caractérise par une altération du collagène et des fibres élastiques, mettant en évidence des changements structurels.

Au cours du temps, l'érythème généré par la vergeture rosée entre dans la phase chronique cicatricielle, de sorte que la vergeture rosée se transforme en vergeture blanche. Celle-ci devient visible sous la forme d'un tissu atrophique, générant une marque permanente ridée, souvent linéaire, hypo-pigmentée, déprimée et flasque, qui est visuellement proche d'une cicatrice.

Les vergetures frappent les femmes avec une fréquence deux fois plus élevée que celle des hommes, elles peuvent apparaître à tout âge mais surtout durant la puberté ou la grossesse. En effet, durant la grossesse environ 44 à 88% des femmes sont touchées.

Les principaux états physiologiques qui peuvent provoquer la réapparition de vergetures sont les suivants : la puberté, la grossesse, la pratique plus ou moins intensive de sports pour développer les muscles, la perte et/ou l'augmentation rapide de poids. Une distension mécanique induite par un traumatisme rapide et soudain, qu'il soit faible ou répété, peut également provoquer la formation de vergetures.

Le mécanisme physiopathologique de la formation des vergetures semble dû à des causes attribuables principalement à un facteur biochimique et hormonal ou encore un facteur d'étirement mécanique, soit une mise en tension exagérée de la peau due à une variation soudaine à la fois en excès et en défaut du pannicule adipeux ou de certaines parties du corps tels que l'abdomen, les fessiers ou les cuisses.

Les microtraumatismes locaux répétés ou l'allongement progressif de la peau provoquent également un étirement des capillaires dermiques, avec pour conséquence une réduction du flux sanguin vers les structures cutanées et une ischémie de zone conséquente ; la réduction de l'apport en oxygène et en substances nutritives provoque une souffrance métabolique qui augmente l'atrophie.

L'aspect histologique des vergetures diffère selon leur phase de développement. En effet, dans les phases précliniques et les lésions initiales, l'épiderme n'apparaît pas modifié. Les altérations biochimiques de la substance fondamentale et de la composante fibreuse, qui créent les conditions de formation des vergetures, sont révélées au niveau du procollagène de type I et III et de la fibronectine.

Aujourd'hui, les vergetures ne peuvent plus être considérées comme des lésions indélébiles même si la possibilité d'une régression reste souvent limitée et en tout cas conditionnée par la période de formation des vergetures et le stade de leur processus de cicatrisation.

Plusieurs traitements contre les vergetures existent à ce jour, sans distinction entre les vergetures rosées et blanche, pourtant différentes histologiquement et biochimiquement. Le manque de spécificité des traitements existants, en fonction du type de vergeture, fait que ces traitements existants ne sont pas satisfaisants.

Néanmoins, ces techniques présentent plusieurs effets secondaires indésirables, dont l'hyperpigmentation post inflammatoire, l'hyperpigmentation transitoire, la réapparition d'érythème, d'oedème, d'intolérance cutanée ou d'ecchymose.

Sur le marché actuel, on distingue la technique physique et la technique chimique de traitement des vergetures.

Parmi les techniques mécaniques, on citera par exemple la micro- dermabrasion, la radiofréquence, le traitement par laser fractionné, laser à diode, laser excimère au chlorure de xénon, laser vasculaire, la lumière pulsée intense, l'infrarouge, la lumière UV, mais aussi la thérapie percutanée d'induction de collagène, ou du plasma riche en plaquette.

Parmi les techniques chimiques par application topique, on citera par exemple le peeling chimique, impliquant l' application d'acide trichloracétique ou d'acide glycolique. Le peeling chimique présente, en outre, des effets secondaires indésirables comme une hyperpigmentation postinflammatoire. Parfois, pour augmenter son efficacité, le peeling chimique à l'acide glycolique est réalisé en combinaison avec de la trétinoïne, qui est déconseillée pendant la grossesse et l'allaitement. Ainsi, le peeling chimique à l'acide glycolique combiné à la trétinoine ne permet pas de traiter tous les sujets avec vergeture.

D'autres composés chimiques, d'application topique associée à un massage cutané, sont connus pour traiter les vergetures en général. On citera par exemple des crèmes contenant de l'acide hyaluronique ou des extraits de Centella asiatica, l'application d'huile d'amande douce ou d'huile d'olive. Toutefois, à ce jour, l'action chimique sur les vergetures n'a pas été dissociée de l'effet mécanique lié au massage répété de la peau.

Le document CN 104 367 522 divulgue une poudre cosmétique pour éliminer les vergetures liées à la grossesse (i.e., stria gravidarum). La composition comprend de la poudre de tomate (Solanum lycopersicum), des germes de blé, de la poudre d'amandes, etc... Selon ce document, le germe de blé assure la réparation des tissus cutanés et éclaircit les taches et rides/stries (i.e., vergetures).

Le document CN 106 137 854 divulgue un gommage pour éclaircir/réduire les vergetures comprenant notamment des particules de fibres de tomate, des particules de fibres de citron, etc.. Il est indiqué que le lycopène contenu dans les tomates prévient les dommages des radicaux libres, inhibe la peroxydation lipidique et a des propriétés anti-taches de rousseur, beauté, anti-âge, soins de la peau, etc., permettant, in fine, la dilution et l'élimination des vergetures. Il est à noter que le lycopène appartient à la famille des caroténoïdes et est connu de l'homme du métier pour son activité de piégeur de radicaux libres (i.e., antioxydant) et son effet anti-inflammatoire via l'inhibition du facteur nucléaire kappa-B (NF-κB).

Le document CN 106 281 808 divulgue un savon pour réduire/éclaircir les vergetures comprenant de l'huile de camélia, du jus de tomate, du pollen de pin, etc.. Il est précisé que le jus de tomate contient du lycopène assurant les mêmes effets que ceux décrits dans ci-dessus.

Le document WO 2008/080443 divulgue une composition cosmétique pour le traitement et/ou la prévention des vergetures comprenant au moins un principe actif choisi parmi l'extrait d'aloe vera. Ce document mentionne que les matrikines, la rutine et l'extrait de Phaseolus lunatus agissent en synergie pour traiter et/ou prévenir les vergetures. Il mentionne en outre que les propriétés anti-inflammatoires et antioxydantes de l'aloe vera peuvent être principalement attribuées à la présence d'isoenzymes de la superoxyde dismutase.

Les isoenzymes sont des enzymes qui catalysent la même réaction chimique la SOD mais présentent des structures différentes et des propriétés chimiques et physiques différentes.

Le document XP055952669 divulgue une crème pour traiter les vergetures comprenant notamment une superoxyde dismutase. Ce produit est conçu pour apporter du soin aux peaux tendues et présente un effet de réduction des rides/ridules.

Le document WO 2007/000619 divulgue l'utilisation d'un mélange de superoxyde dismutase et de catalase pour le traitement de lésions inflammatoires de la peau, en particulier les fibroses du derme, les kératoses épidermiques, les cicatrices chéloïdes ou les escarres hypertrophiques.

Ainsi, il est nécessaire de disposer de nouvelles compositions efficaces pour le traitement et/ou la prévention de la réapparition des vergetures rosées spécifiquement.

### Exposé de l'invention

L'un des buts de l'invention est donc de proposer une composition pour le traitement et/ou la prévention de la réapparition spécifiquement des vergetures rosées capable de répondre aux inconvénients précités.

À cet effet, il a été mis au point une composition comprenant, en tant que principe actif dermatologique, une superoxyde dismutase, pour son utilisation dans le traitement et/ou la prévention de la réapparition des vergetures rosées.

On entend par « superoxyde dismutase (SOD) », les superoxydes dismutases 1, 2 et 3, prises séparément ou en combinaison.

On entend par le terme « principe actif dermatologique », un composé destiné à être appliqué sur la peau et qui possède des propriétés biologiques qui sous -tendent un effet physiologique. Le principe actif est à différencier des « excipients » de la composition.

On entend par le terme « excipient », tous composés qui confèrent à la composition ses propriétés physicochimiques, telles que sa consistance, sa galénique et sa capacité à délivrer ledit principe actif dermatologique en étant inerte vis-à-vis de son effet physiologique.

On entend par l'expression « prévention de la réapparition des vergetures rosées », le fait d'éviter la réapparition de nouvelle vergeture rosée à la surface de la peau.

On entend par l'expression « traitement des vergetures rosées », le fait de limiter leur développement, leur croissance et leur étendue à la surface de la peau.

En d'autres termes, l'invention a pour objet une superoxyde dismutase, avantageusement stabilisée pour son utilisation dans le traitement et/ou la prévention de la réapparition des vergetures rosées.

Selon une forme de réalisation préférée de la composition de l'invention, la superoxyde dismutase est stabilisée.

Avantageusement, la superoxyde dismutase est d'origine végétale, de préférence extraite de la tomate (*Solanum Lycopersicum*).

La superoxyde dismutase s'est avérée posséder un effet anti inflammatoire et un effet antifibrotique, essentiels pour le traitement des vergetures rosées.

La superoxyde dismutase, sous la forme d'un extrait de tomate notamment a en outre pour avantage d'être facilement mise en formulation, d'être non cytotoxique, biocompatible et acceptée par la règlementation des produits cosmétiques en vigueur.

Selon un mode de réalisation préféré, l'extrait de tomate est stabilisé, en particulier par encapsulation au moyen d'une solution comprenant de la maltodextrine, avantageusement d'une solution contenant uniquement de la maltodextrine. En pratique, l'encapsulation est opérée par séchage par pulvérisation (spray drying).

Dans un mode de réalisation préféré, la solution comprenant de la maltodextrine représente entre 20% et 50% en poids de l'extrait de tomate.

De préférence, la composition comprend comme seul principe actif dermatologique, ladite superoxyde dismutase.

Selon un mode de réalisation préféré de la composition de l'invention, la superoxyde dismutase est extraite de la tomate entière.

Selon un mode préféré de réalisation de la composition de l'invention, l'extrait de tomate est stabilisé au moyen d'une solution comprenant de la maltodextrine, avantageusement uniquement de la maltodextrine, l'ensemble représentant entre 1% et 10% en poids total de la composition, avantageusement entre 1% et 5%.

Selon un mode de réalisation préféré, la composition utilisée pour traiter et/ou prévenir les vergetures rosées, se présente sous une forme galénique adaptée pour un usage topique. En d'autres termes, ladite composition est sous forme galénique qui permet au principe actif dermatologique d'agir localement, en ciblant les vergetures rosées, en particulier à l'endroit d'application de la composition sur la peau.

De préférence, la composition utilisée pour traiter et/ou de prévenir la réapparition des vergetures rosées, se présente sous la forme d'une émulsion, d'un gel, d'une crème, d'un lait, d'une huile, d'une lotion, d'une solution, d'une pommade, ou encore d'une mousse. Cette forme galénique a pour avantage de permettre une application facile et ciblée sur les vergetures rosées.

Selon un mode de réalisation particulier, la composition selon l'invention est une composition cosmétique.

En d'autres termes, l'invention concerne donc une utilisation cosmétique d'une superoxyde dismutase, en tant que principe actif dermatologique, pour traiter et/ou de prévenir la réapparition des vergetures rosées.

Selon un mode de réalisation préféré, la composition utilisée pour traiter et/ou de prévenir la réapparition des vergetures rosées, se présente sous une forme galénique adaptée pour un usage topique.

En d'autres termes, ladite composition est sous une forme galénique qui permet au principe actif dermatologique d'agir localement, en ciblant les vergetures rosées, en particulier à l'endroit d'application de la composition sur la peau.

De préférence, la composition utilisée pour traiter et/ou de prévenir la réapparition des vergetures rosées, se présente sous une forme galénique adaptée pour une voie d'administration cutanée. En d'autres termes, la composition est administrée à la surface de la peau, dans ce cas, la forme galénique peut être par exemple une crème, un gel, une huile, une lotion, une pommade, une mousse.

Selon un autre mode de réalisation, la composition, utilisée pour traiter et/ou de prévenir la réapparition des vergetures rosées, se présente sous une forme galénique adaptée pour une voie d'administration transcutanée. En d'autres termes, la composition est appliquée à travers la peau, au cours d'une injection sous-cutanée, par exemple avec une aiguille contenant la composition sous forme liquide, par exemple sous forme d'une solution.

Pour améliorer les propriétés hydratantes de la composition, celle-ci contient en outre de l'huile d'avocat, représentant en particulier entre 0,5% et 5% du poids total de la composition.

La présente invention concerne également un procédé cosmétique, pour le traitement et/ou la prévention de la réapparition des vergetures rosées, selon lequel on applique sur les vergetures rosées une quantité efficace d'une composition selon l'invention.

On entend par l'expression « quantité efficace », la quantité nécessaire, de la composition, à appliquer sur les vergetures rosées, pour obtenir un effet physiologique significatif qui permet de traiter et/ou de prévenir la réapparition des vergetures rosées.

Dans le cas d'une application de la composition de l'invention par voie topique, on masse légèrement la peau au moment de l'application de la composition ce qui favorise sa pénétration dans les différentes couches de la peau afin notamment que le principe actif dermatologique, à savoir, la superoxyde dismutase, atteigne l'épiderme, le derme, voire l'hypoderme. Dans le cas d'une application de la composition par voie transcutanée, l'injection ciblée de la composition selon l'invention, permet de libérer directement la superoxyde dismutase dans l'épiderme, le derme, voir l'hypoderme.

Selon un mode de réalisation préféré du procédé cosmétique de l'invention, on nettoie préalablement la peau, puis on applique deux fois/jour, de préférence 1 fois le matin et 1 fois le soir, la composition sur les vergetures rosées.

Selon un mode de réalisation privilégié, on utilise entre 0,02 et 0,04 millilitre de la composition par cm² de peau par application.

En conséquence, la composition de l'invention permet d'éviter la formation et le développement des vergetures rosées. L'usage de la composition de l'invention permet d'éliminer l'aspect inesthétique des vergetures rosées et de réduire leurs croissances et/ou leur extension sur la peau.

### Brève description des dessins

D'autres avantages et caractéristiques de l'invention ressortiront mieux de la description qui va suivre, donnée à titre d'exemple non limitatif, de la composition selon l'invention, à partir des dessins annexés dans lesquels :

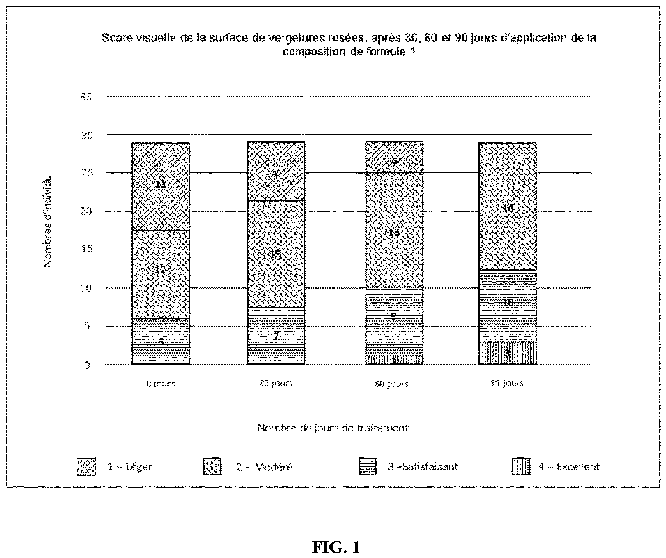

La Figure 1 représente, l'évaluation de l'amélioration de la surface dermique des vergetures rosées, observée après 30 jours, 60 jours ou 90 jours d'application par voie cutanée de la composition selon l'invention.

### Description détaillée de l'invention

Selon un mode de réalisation préférée, la composition selon l'invention, pour son utilisation dans le traitement et/ou la prévention de la réapparition de vergetures rosées est reproduite dans le tableau 1.

**[Tableau 1] :**

| Composant | % du poids total de la composition |
|---|---|
| Eau | 71,605 |
| Propylène glycol dipélargonate | 6 |
| Propylène glycol | 5 |
| Stéarate de glycérol et du PEG-75 stéarate | 4 |
| Acide stéarique | 3 |
| Huile minérale | 2 |
| Huile d'avocat | 1 |
| Extrait de tomate stabilisé par une solution de maltodextrine | 2 |
| Palmitate de cétyle | 2 |
| Stéarate de glycol | 1 |
| Pétrolatum | 0,5 |
| Tri-éthanolamine | 0,325 |
| Méthyl parabène | 0,300 |
| Acide sorbique | 0,300 |
| Silicate de magnésium et d'aluminium | 0,300 |
| Gomme de cellulose | 0,300 |
| Gomme de xanthane | 0,300 |
| Hydroxypropyltrimonium maltodextrine cross polymère | 0,035 |
| Chlorure de sodium | 0,020 |
| Glycolate de sodium | 0,015 |

Dans le tableau 1 ci-dessus, tous les composants sont considérés comme des excipients de formulation, sauf l'extrait de tomate riche en superoxyde dismutase qui consiste en le seul principe actif dermatologique.

### Evaluation de l'efficacité et de la tolérance de l'application d'une composition cosmétique selon la formule 1 pour traiter les vergetures rosées

Pour évaluer l'efficacité et la tolérance d'application de la composition de l'invention, 29 individus ont appliqué, matin et soir, pendant 90 jours, sur une zone de peau nettoyée présentant des vergetures rosées, soit une composition selon l'invention, soit une composition « placebo ».

Dans le protocole d'étude, les individus sont constitués d'hommes (1) et de femmes (28). Pour chacun d'entre eux, aucun traitement des vergetures rosées n'a été réalisé dans les 4 semaines avant le démarrage du protocole d'étude, afin d'éviter toutes interactions ou effets liés à des traitements antérieurs.

La composition cosmétique selon l'invention est de formule 1. La composition « placebo » est de formule identique à la formule 1, mais sans le principe actif (SOD).

Chez chaque individu, la composition « placebo » a été appliquée sur une première moitié de la zone constituée par les vergetures rosées, et la composition de l'invention sur l'autre moitié de ladite zone. L'application cutanée sur les différentes zones de vergetures rosées s'est accompagnée d'un léger massage pour favoriser la pénétration dans la peau de chacune des deux compositions. En moyenne, il a été appliqué entre 0,02 et 0,04 millilitre de composition par cm² de peau.

L'évolution de la surface des vergetures rosées a été évaluée après 30 jours, 60 jours et 90 jours d'applications, pour la zone traitée avec la composition de l'invention par rapport à la zone traitée avec la composition « placebo ».

Le tableau 2 ci-dessous montre le pourcentage moyen d'amélioration observée de la surface de chacune des vergetures rosées évaluées, en fonction du nombre de jours de traitement et pour chacune des compositions testées.

L'analyse de la surface des vergetures rosées est une analyse planimétrique, réalisée avec le logiciel ImageJ, qui permet, au cours du temps, d'évaluer l'étendue de la surface d'une vergeture rosée prédéterminée, par rapport à sa surface initiale de départ pré-protocole. La surface initiale de départ qu'occupe une vergeture déterminée sur la peau correspond à une surface de référence définie comme étant de 100%.

Une diminution de la surface de la vergeture rosée déterminée, au cours du temps, se traduit par un pourcentage d'amélioration de la lésion dermique qui est positif et croissant.

Au contraire, une augmentation de la surface de la vergeture rosée déterminée, au cours du temps, se traduit par un pourcentage d'amélioration de la lésion dermique qui est décroissant, faible, voire négatif

**[Tableau 2] :**

| | % d'amélioration de la surface des vergetures rosées | |
|---|---|---|
| Nombre de jours de traitement | Avec la composition de l'invention de formule 1 | Avec la composition " placebo" |
| 30 jours | 8,14% | 3.59% |
| 60 jours | 21,00% | -2.28% |
| 90 jours | 34,90% | -0,09% |

Le tableau 2 montre que l'application de la composition de formule 1 de l'invention améliore significativement la lésion des vergetures rosées, alors que ceci n'est pas le cas avec la composition « placebo »

Les résultats montrent par exemple qu'après 90 jours de traitement avec la composition selon l'invention, en moyenne sur l'ensemble des vergetures rosées initiales considérées, les vergetures rosées perdent en moyenne 35% de leur surface, et jusqu'à 73,5% de leur surface par rapport à la surface qu'elles occupaient initialement sur la peau avant le traitement. L'usage de la composition de l'invention entraine donc une diminution significative de l'étendue des vergetures rosées sur la peau. Ainsi, la présence de la superoxyde dismutase joue un rôle de principe actif dermatologique dans la composition de l'invention en améliorant l'état et l'étendue de la surface occupée par les vergetures rosées.

Il est à noter qu'aucun effet secondaire n'a été observé, pendant toute la période d'application de la composition de formule 1. On entend par « effet secondaire » des brûlures, irritations, démangeaisons ou tout autres types de modification de la peau au niveau de la surface d'application de la composition de l'invention. Ainsi, la superoxyde dismutase, en tant que principe actif dermatologique, en association avec les excipients, est bien tolérée en application topique.

En outre, une évaluation visuelle de la lésion sur la zone traitée par la composition de l'invention a été réalisée sur chaque individu. L'évaluation visuelle, après 30, 60 et 90 jours d'application, est déterminée en indiquant un score allant de 1 à 4 pour l'aspect visuel de la vergeture rosée.

Par le terme « aspect visuel », on entend désigner la longueur, la largeur, l'intensité de la couleur rosée, la surface totale d'une vergeture rosée.

Le score 1 signifie une légère amélioration visuelle de l'aspect de la vergeture rosée, de l'ordre de 0 à 25%, par rapport à la vergeture rosée initiale sans traitement.

Le score de 2 signifie une amélioration visuelle modérée, de l'ordre de 25 à 50% par rapport à l'aspect initial de la vergeture rosée sans traitement.

Le score de 3 signifie une bonne amélioration visuelle, de l'ordre de 50 à 75% par rapport à l'aspect initial de la vergeture rosée sans traitement.

Le score de 4 signifie une excellente amélioration visuelle, supérieure à 75% par rapport à l'aspect initial de la vergeture rosée sans traitement.

La figure 1 récapitule les scores évalués pour chaque individu, en fonction du nombre de jours de traitement, après application, matin et soir, de la composition de l'invention de formule 1 sur une zone de peau présentant des vergetures rosées.

Les résultats montrent qu'après 30 jours d'application de la composition de l'invention, ¼ des patients présentent une amélioration visuelle légère (7 individus), plus de la moitié des patients (15 individus) présentent une amélioration modérée de la lésion dermique de la vergeture rosée évaluée.

De la même manière, après 60 jours de traitements, le nombre de patients présentant une amélioration dermique modérée (15 individus) de la lésion est inchangé, alors que celui des patients présentant une amélioration satisfaisante (9 individus) a augmenté de 1/3 comparé au début de l'étude clinique. De plus, un individu présente une excellente amélioration visuelle de la lésion des vergetures rosées.

Après 90 jours de traitement, plus de la moitié des personnes (16 individus) présente une amélioration modérée des vergetures, 1/3 présente une amélioration satisfaisante des vergetures et trois personnes présentent une excellente amélioration de la lésion des vergetures rosées avec un score de 4.

En d'autres termes, les résultats de la figure 1 montrent, qu'au cours du temps, l'application de la composition de formule 1 selon l'invention, améliore le score visuel de l'état de surface de la lésion dermique à l'origine de la vergeture rosée.

Ainsi, la composition de l'invention a pour effet d'améliorer l'état et l'aspect visuel des vergetures rosées existantes. La composition de l'invention permet également de prévenir la réapparition de nouvelles vergetures rosées, mais aussi de ralentir le développement de celles existantes, en diminuant la surface globale qu'elles occupent sur la peau.

En plus de son efficacité démontrée même à faible concentration, la superoxyde dismutase, en tant que principe actif dermatologique d'une composition pour les vergetures rosées, est facile à formuler.

L'usage d'une superoxyde dismutase dans une composition pour traiter et/ou de prévenir la réapparition des vergetures rosées est également facile à mettre en oeuvre et peu coûteux.

Ainsi, la composition de l'invention, pour traiter et/ou de prévenir la réapparition des vergetures rosées, comprenant en tant que principe actif dermatologique, une superoxyde dismutase consiste en une solution efficace, peu coûteuse, utilisable par tout type de sujet, facile à mettre en oeuvre pour traiter et/ou de prévenir la réapparition des vergetures rosées de manière ciblée.

## Revendications

1. Superoxyde dismutase, pour utilisation dans le traitement et/ou la prévention de la réapparition des vergetures rosées.

2. Superoxyde dismutase pour utilisation, selon la revendication 1, **caractérisée en ce qu'**elle est stabilisée.

3. Superoxyde dismutase pour utilisation, selon la revendication 1, **caractérisée en ce qu'**elle est d'origine végétale.

4. Superoxyde dismutase pour utilisation, selon la revendication 3, **caractérisée en ce qu'**elle est extraite de tomate (*Solanum Lycopersicum*).

5. Superoxyde dismutase pour utilisation, selon la revendication 4, **caractérisée en ce que** l'extrait de tomate est stabilisé par encapsulation au moyen d'une solution comprenant de la maltodextrine.

6. Superoxyde dismutase pour son utilisation selon la revendication 5, **caractérisée en ce que** la solution comprenant de la maltodextrine représente entre 20% et 50% en poids de l'extrait de tomate.

7. Superoxyde dismutase, pour utilisation selon l'une des revendications 3 à 6, **caractérisée en ce qu'**elle est extraite de la tomate entière.
